(19) Europäisches Patentamt
European Patent Office
Office européen des brevets

(11) Veröffentlichungsnummer: **0 085 030**
**B1**

(12) # EUROPÄISCHE PATENTSCHRIFT

(45) Veröffentlichungstag der Patentschrift: **22.05.85**

(51) Int. Cl.[4]: **C 07 D 213/30**, C 07 D 213/26, C 07 D 213/16, C 07 D 213/57, C 07 F 9/58

(21) Anmeldenummer: **83810026.1**

(22) Anmeldetag: **21.01.83**

(54) **Verfahren zur Herstellung von substituierten Divinylpyridinen und neue substituierte Divinylpyridine.**

(30) Priorität: **27.01.82 CH 501/82**

(43) Veröffentlichungstag der Anmeldung: **03.08.83 Patentblatt 83/31**

(45) Bekanntmachung des Hinweises auf die Patenterteilung: **22.05.85 Patentblatt 85/21**

(84) Benannte Vertragsstaaten: **CH DE FR GB IT LI**

(56) Entgegenhaltungen: **DE - A - 2 615 309**
**US - A - 2 739 948**

(73) Patentinhaber: **CIBA-GEIGY AG, Postfach, CH-4002 Basel (CH)**

(72) Erfinder: **Kaschig, Jürgen, Dr., 29 Major Appleby's road, Ardsley New York 10502 (US)**

EP 0 085 030 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von in 6-Stellung substituierten Divinylpyridinen sowie die dadurch herstellbaren neuen Divinylpyridine und Zwischenprodukte.

Gegebenenfalls in 4- oder 6-Stellung durch eine Methylgruppe substituierte Divinylpyridine können z. B. dadurch hergestellt werden, dass man Dimethyl- oder Trimethylpyridine durch Erhitzen mit Formaldehyd in die entsprechenden Bis-β-hydroxyäthylpyridine überführt und diese dehydratisiert. Auf analoge Weise können Methylvinyl- oder Methyläthylpyridine zu Divinylpyridinen umgesetzt werden, wobei im Falle von Methyläthylpyridinausgangsprodukten das nach der Dehydratisierung erhaltene Vinyläthylpyridin noch katalytisch dehydriert werden muss. Auf diese Weise wurden neben unsubstituierten Divinylpyridinen auch das 2,6-Divinyl-4-methyl- und 2,4-Divinyl-6-methylpyridin hergestellt (vgl. z. B. „Bull. Acad. Polon. Sci.", „Sér. Sci. chim.", 8, 217 [1960]; „Roczniki chem.", *29*, 141 [1955], *38*, 1337 [1964] und *40*, 1505 [1966]; „Zhur. Prikl. Khimii", *45* [4], 872 [1972], US-PS Nr. 2739948, russ. PS Nr. 234407, FR-PS Nr. 1525475 und DDR-PS Nr. 54361). Unsubstituierte Divinylpyridine können auch bei Temperaturen zwischen etwa 450 und 800°C durch katalytische Dehydrierung von Diäthyl- oder Äthylvinylpyridinen erhalten werden (vgl. z. B. US-PS Nrn. 2728770 und 2611769, DE-OS Nr. 2061241). Schliesslich kann 2,5-Divinylpyridin auch durch Manich-Reaktion aus 2-Methyl-5-vinylpyridin hergestellt werden (vgl. z. B. „Khim. Geterotsikl. Soedin.", *4*, 667 [1967]). Bei diesen vorbekannten Verfahren sind die Ausgangsprodukte zum Teil schwer zugänglich, vor allem aber sind die Ausbeuten an Divinylpyridinen unbefriedigend (meistens unter 10%, bezogen auf das Pyridinausgangsprodukt).

In der DE-A Nr. 2615309 ist offenbart, wie man aus Nitrilen und Acetylen 2-substituierte Pyridine unter Verwendung von Kobaltocen herstellt. Es ist aber nicht beschrieben, wie in 6-Stellung substituierte Divinylpyridine bzw. Vorläufer synthetisiert werden können.

Es wurde nun ein neues Verfahren gefunden, nach dem sich in 6-Stellung substituierte Divinylpyridine und Vorläufer davon unter relativ milden Reaktionsbedingungen und unter Verwendung von leicht zugänglichen und billigen Ausgangsprodukten in guten bis sehr guten Ausbeuten herstellen lassen. Gegenstand der Erfindung ist somit ein Verfahren zur Herstellung von Verbindungen der Formel I:

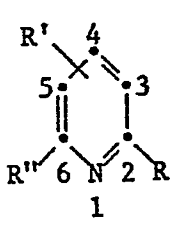

(I)

worin R' in 4- oder 5-Stellung gebunden ist,

R und R' $-CH_2OH$, $-CH_2CH_2OH$, $-CH_2CH_2OCH_2C_6H_5$, $-CH_2-X$, $-CH_2P^+(Y)_3X^-$, $-CH_2P(O)(C_{1-3}\text{-Alkoxy})_2$ oder $-CH=CH_2$,

R'' $C_{1-10}$-Alkyl,

X ein Halogenatom, wie Chlor, Brom oder Jod, besonders Chlor und vor allem Brom,

$X^-$ das X entsprechende Anion, und

Y Phenyl, das durch eine $C_{1-5}$-Alkylgruppe substituiert sein kann, bedeuten,

indem man eine Verbindung der Formel II:

$$R''-CN \qquad (II)$$

bei Temperaturen zwischen 40 und 180°C in Gegenwart eines Kobalt(I)-Katalysators mit einer Verbindung der Formel III:

$$HC\equiv C-Z \qquad (III)$$

zu Verbindungen der Formel IV:

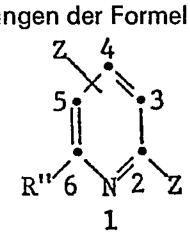

(IV)

umsetzt, wobei R'' die unter Formel I angegebene Bedeutung hat und die Z $-CH_2OH$, $-CH_2CH_2OH$ oder $-CH_2CH_2OCH_2C_6H_5$ bedeuten, Verbindungen der Formel IV, worin die Z $-CH_2CH_2OH$ oder $-CH_2CH_2OCH_2C_6H_5$ darstellen, gegebenenfalls unter vorheriger Hydrierung von Gruppen Z = $-CH_2CH_2OCH_2C_6H_5$ zu Gruppen $-CH_2CH_2OH$, zu Verbindungen der Formel I mit R und R' = $-CH=CH_2$ dehydratisiert, und Verbindungen der Formel IV mit Z = $-CH_2OH$ durch Behandlung mit einem Halogenierungsmittel zu Verbindungen der Formel V:

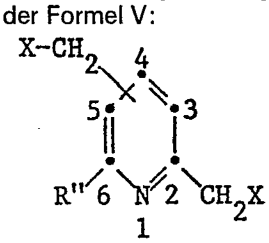

(V)

umsetzt, die Verbindungen der Formel V mit einer Verbindung einer der Formeln VIa oder VIb:

$$P(Y)_3 \qquad (VIa)$$

oder

$$P(O)(C_{1-3}\text{-Alkoxy})_3 \qquad (VIb)$$

zu Verbindungen einer der Formeln VIIa oder VIIb

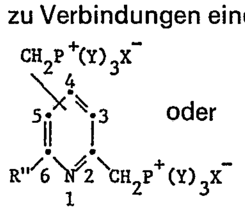

oder

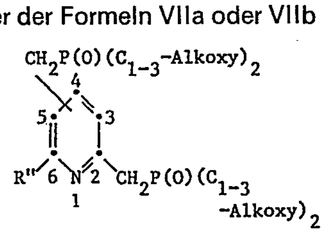

(VIIa) (VIIb)

umsetzt und die Verbindungen einer der Formeln VIIa oder VIIb in Gegenwart einer Base mit Formaldehyd zu Verbindungen der Formel I mit R und R' = $-CH=CH_2$ umsetzt (Wittig- bzw. Wittig-Horner-Reaktion). Dabei haben R'', Y, X und $X^-$ die unter Formel I angegebene Bedeutung und die zweite Gruppe Z, $-CH_2$Halogen, $-CH_2P^+X^-$ oder $-CH_2P(O)(C_{1-3}\text{-Alkoxy})_2$ ist jeweils in 4- oder 5-Stellung an den Pyridinring gebunden.

Dabei ist es überraschend, dass sich Nitrile der Formel II in Gegenwart von Kobalt(I)-Katalysatoren, d. h. starken Lewis-Säuren, mit OH-Gruppen aufweisenden Alkinen der Formel III zu Pyridinderivaten der Formel IV cyclotrimerisieren lassen. Es ist nämlich bekannt, dass die Umsetzung von Nitrilen mit Alkoholen, u. a. Propargylalkohol, in Gegenwart von sauren oder basischen Katalysatoren, wie HCl, HBr, Natriummethoxid oder Natriumäthoxid, zu Iminoäthern führt. Diese können in Gegenwart von Salzen der Metalle der Gruppen Ib, IIb und VIIIb, z. B. Ag(I)- und HG(I)-Salzen, intramolekulär zu Oxazolderivaten cyclisiert werden (vgl. z. B. „Chem. Review", *61*, 179 [1961]; „J. Org. Chem.", *26*, 412 [1961] und DE-OS Nr. 2162367). Nitrile, wie Benzonitril und Acetonitril, können in Gegenwart bestimmter starker Säuren, wie Schwefelsäure, Phosphorsäure und Polyphosphorsäure, mit α-Acetylenalkoholen auch direkt zu Oxazolderivaten umgesetzt werden (vgl. z. B. JP-AS Nr. 29849/64).

Verbindungen der Formel I, worin R und R' $-CH=CH_2$ darstellen und R'' die angegebene Bedeutung hat, können nach einem abgeänderten Verfahren auch dadurch hergestellt werden, dass man eine Verbindung der Formel II bei Temperaturen zwischen 30 und 170, vorzugsweise 100 und 160°C, in Gegenwart eines Kobalt(I)-Katalysators mit Vinylacetylen umsetzt.

Die Umsetzung wird vorzugsweise unter Durchflussbedingungen durchgeführt. Nach diesem abgeänderten Verfahren entstehen die Divinylpyridine überraschenderweise als Hauptprodukte, obwohl die Umsetzung von konjugierten Acetylenen, wie Vinylacetylen, Methylpropiolat und Phenylacetylen, mit Nitrilen in Gegenwart von Kobaltkatalysatoren gemäss US PS Nr. 3829429 vornehmlich Oligomerisationsprodukte der konjugierten Acetylene liefert. Die Herstellung von in 6-Stellung substituierten Divinylpyridinen ist in der genannten US PS nicht konkret beschrieben.

Erfindungsgemäss anfallende Isomerengemische können gewünschtenfalls auf übliche Weise getrennt werden, z. B. durch Kristallisation. Alkylgruppen R'', Alkoxygruppen in Resten R und R' sowie Alkylsubstituenten an Phenylgruppen Y können geradkettig oder verzweigt sein. Als Beispiele von Alkylgruppen R'' seien genannt: Methyl, Äthyl, n-Propyl, Isopropyl, n-Butyl, sek.-Butyl, n-Pentyl, 2-Pentyl, n-Hexyl, 2-Äthylhexyl, n-Heptyl, n-Octyl, n-Nonyl und n-Dodeyl. Bevorzugt stellt R'' geradkettiges oder verzweigtes Alkyl mit 1 bis 4 C-Atomen und insbesondere Methyl dar.

Beispiele von Alkoxygruppen in Resten R und R' sind Methoxy, Äthoxy, n-Propyloxy und Isopropyloxy. Bevorzugt sind Äthoxy und Isopropyloxy.

Sind Phenylgruppen Y durch $C_{1-5}$-Alkyl substituiert, so handelt es sich z. B. um Methyl-, Äthyl-, n-Propyl-, Isopropyl-, n-Butyl- und n-Pentylgruppen, vor allem Methyl- oder Äthylgruppen. Besonders bevorzugt ist Y jedoch unsubstituiertes Phenyl.

X ist vorzugsweise Chlor und insbesondere Brom. Z ist bevorzugt $-CH_2CH_2OH$ und insbesondere $-CH_2OH$. Die Verbindungen der Formel IV und deren Folgeprodukte einer der Formeln V, VIIa oder VIIb fallen im allgemeinen als Isomerengemische an. Isomerengemische von Verbindungen der Formel IV mit Z = $-CH_2OH$ und die daraus erhältlichen Folgeprodukte können auf besonders einfache Weise, z. B. durch Kristallisation in geeigneten Lösungsmitteln oder Lösungsmittelgemischen, wie Aceton/Diäthyläther oder N,N-Dimethylformamid, in die einzelnen Isomeren aufgetrennt werden.

Als Kobalt(I)-Katalysatoren für die Cocyclotrimerisierung der Nitrile der Formel II mit den Alkinen der Formel III sowie im abgeänderten Verfahren zur direkten Herstellung von Divinylpyridinen der Formel I können an sich bekannte Verbindungen eingesetzt werden, wie sie z. B. in „Synthesis, Communications", 575 [1974] und 26 [1976] sowie in der US-PS Nr. 4006149 und der DE-OS Nr. 2742541 beschrieben sind.

Geeignete Katalysatoren sind beispielsweise Cyclopentadienyl/Kobaltcyclopentadien (Kobaltocen), das an einer Cyclopentadienylgruppe auch durch $-CH_2CN$, $-CH(CN)-CH_3$, $-C(CN)=CH_2$ oder $-CH(CN)CH_2OCH_3$ substituiert sein kann, Cyclopentadienyltetraphenyl/Kobaltcyclopentadien, Cyclopentadienyl/Kobaltcyclooctadien, $\eta^3$-Cyclooctadienyl/Kobaltcyclooctadien, Methylheptadienyl/Kobaltbutadien. Die Kobaltkatalysatoren können als solche eingesetzt oder *in situ* durch Reduktion von zwei- oder dreiwertigen Kobaltsalzen anorganischer oder organischer Säuren hergestellt werden. Als Kobaltsalze kommen z. B Kobalt(II)chlorid, -fluorid, -carbonat, -hydroxidcarbonat, -sulfat, -nitrat, -formiat, -acetat, -acetylacetonat, -äthylat, -n-butylat, -oxalat, -phenolat und -naphthenat sowie Kobalt(III)acetylacetonat in Betracht. Als Reduktionsmittel eignen sich Metalle der Gruppen I bis III des Periodischen Systems und vor allem deren Hydride, wie Lithium, Natrium, Magnesium, Calcium, LiH, $LiAlH_4$ und $NaBH_4$. Bevorzugtes Reduktionsmittel ist $NaBH_4$. Besonders bevorzugt verwendet man im erfindungsgemässen Verfahren Cyclopentadienyl/Kobaltcyclooctadien als Katalysator.

Der Katalysator wird zweckmässig in Mengen von 0,1 bis 3, insbesondere 0,1 bis 1 Mol.-%, bezogen auf das Alkin der Formel III, verwendet.

Die Reaktionstemperaturen für die Cocyclotrimerisation liegen vorzusgweise zwischen 80 und 160, vor allem zwischen 140 und 150°C. Gegebenenfalls wird unter Druck gearbeitet.

Die Umsetzung kann in Gegenwart eines inerten organischen Lösungsmittels vorgenommen werden. Als solche eignen sich aliphatische oder aromatische Kohlenwasserstoffe, wie Benzol, Toluol, Xylole, n-Pentan, n-Hexan und n-Heptan; aliphatische oder cyclische Äther, wie Diäthyläther, Diisopropyläther, Methyläthyläther, Tetrahydropyran, Tetrahydrofuran und Dioxan.

Das Nitril der Formel II wird zweckmässig im Überschuss über die stöchiometrisch benötigte Menge eingesetzt, bevorzugt in 5- bis 20fachem Überschuss. Dabei dient das überschüssige Nitril auch als Lösungsmittel.

Die Hydrierung der Verbindungen der Formel IV mit Z = $-CH_2CH_2OCH_2C_6H_5$ zu Verbindungen der Formel IV mit Z = $-CH_2CH_2OH$ wird bevorzugt in Gegenwart eines inerten organischen Lösungsmittels vorgenommen. Geeignete Lösungsmittel sind z. B. Äthanol, Methanol oder Dioxan, vor allem aber saure Medien, wie Äthanol/Chlorwasserstoff-Gemische, Essigsäure oder Trifluoressigsäure. Bevorzugt ist Trifluoressigsäure oder deren Gemisch mit Essigsäure. Die genannte Hydrierung wird mit Vorteil katalytisch durchgeführt. Als Katalysatoren eignen sich z. B. Kupferchromoxid und Nickelkatalysatoren, wie Raney-Nickel, besonders aber Edelmetallkatalysatoren, wie Platin- und Palladiumkatalysatoren, ganz besonders Palladium/Kohlenstoff-Katalysatoren. Die Dehydratisierung von Verbindungen der Formel IV mit Z = $-CH_2CH_2OH$ kann auf an sich bekannte Weise, bevorzugt in Gegenwart von NaOH oder KOH, vorgenommen werden. Vorteilhaft wird sie ohne Lösungsmittel bei Temperaturen zwischen 130 und 200°C und zweckmässigerweise unter Mitverwendung von Polymerisationsinhibitoren, z. B. Dinitrochloranilinen, Phenothiazinderivaten, Diarylaminen, Schwefel, Pikrinsäure, α-Nitroso-β-naphthol, Hydrochinon und Phenolen, wie Ditert.-butyl-p-kresol, durchgeführt.

Als Halogenierungsmittel können bei der Umsetzung mit Verbindungen der Formel IV, worin Z = $-CH_2OH$ ist, z. B. Chlor-, Brom- oder Jodwasserstoffsäure, Phosphoroxichlorid, Thionylchlorid oder Thionylbromid eingesetzt werden. Bevorzugt verwendet man konzentrierte wässerige Chlorwasserstoffsäure und vor allem konzentrierte wässerige Bromwasserstoffsäure. Die Umsetzung erfolgt im allgemeinen bei Rückflusstemperatur.

Die weitere Umsetzung mit den Phosphinen der Formel VIa oder den Phosphorigsäureestern der Formel VIb wird mit Vorteil in Gegenwart eines inerten organischen Lösungsmittels, wie Toluol, Tetrahydrofuran oder N,N-Dimethylformamid, und bei Temperaturen zwischen 50 und 150°C vorgenommen.

Die Umsetzung der Verbindungen der Formeln VIIa und VIIb mit Formaldehyd wird in Gegenwart einer Base und gegebenenfalls in Gegenwart eines Lösungsmittels, eines Phasentransferkatalysators und/oder eines Polymerisationsinhibitors durchgeführt. Als Basen eignen sich z. B. Natriumhydrid, n-Butyllithium, Alkalimetall- und Erdalkalimetallalkoholate, Alkalimetall- und Erdalkalimetallhydroxide, Alkalimetall- und Erdalkalimetallcarbonate sowie Trialkylamine mit je 2 bis 4 C-Atomen in den Alkylgruppen. Bei der Reaktion verwendet man vorzugsweise ein inertes Lösungsmittel, wie Benzol, Chlorbenzol, Toluol, Tetrahydrofuran, Dioxan, N,N-Dimethylformamid, Dichlormethan, Methanol, Äthanol oder Wasser. Bevorzugte Lösungsmittel sind Wasser und besonders Zweiphasensysteme aus Wasser und einem wasserunlöslichen organischen Lösungsmittel, wie Dichlormethan. Als Basen finden bevorzugt Alkalimetallhydroxide oder -carbonate Verwendung. Geeignete Phasentransferkatalysatoren sind z. B. Kronenäther, Kryptanten, Tetraalkylammonium-, Tetraalkylaminium- oder Tetraalkylphosphoniumsalze, wie Tetra-n-butylammoniumhydrogensulfat und -cyanid und Tetra-n-butylaminiumjodid. Als Polymerisationsinhibitoren können solche der vorerwähnten Art verwendet werden.

Die Verbindungen der Formel I sind mit Ausnahme des 2,4-Bis-(β-hydroxyäthyl)-6-methylpyridins und des 2,4-Divinyl-6-methylpyridins neu und ebenfalls Gegenstand der vorliegenden Erfindung. Dabei gilt in bezug auf bevorzugte Bedeutungen von R, R', R'', X und Y das vorstehend Angegebene.

Nach dem erfindungsgemässen Verfahren lassen sich in 6-Stellung substituierte Divinylpyridine und die Zwischenprodukte der Formeln IV, V, VIIa und VIIb erstmals in wirtschaftlichen Ausbeuten und teilweise auch auf besonders einfache Weise in isomerenfreier Form herstellen, was bei gewissen Weiterverwendungen von Vorteil sein kann. Divinylpyridine der Formel I können z. B. zur Herstellung von Polymeren (Ionen-Austausch-Harzen; vgl. z. B. US-PS Nrn. 2739948 und 2824844) oder als Vernetzungsmittel eingesetzt werden. Besonders bevorzugt ist das 2,5-Divinyl-6-methylpyridin, das – im Gegensatz zum vorbekannten Isomeren – auch zur Herstellung von wertvollen optischen Aufhellern des Stilbentyps verwendet werden kann, z. B. indem man es wie in den Beispielen beschrieben mit Brombenzonitrilen umsetzt.

*Beispiel 1:*

*2,4-Bis(hydroxymethyl)-6-methylpyridin und 2,5-Bis(hydroxymethyl)-6-methylpyridin*

a) *Herstellung unter Verwendung eines Druckreaktors.*

Eine Lösung aus 492 g (12 mol) Acetonitril, 168 g (3 mol) Propargylalkohol sowie 4,5 g (0,0195 mol) Cyclopentadienyl/Kobaltcycloocta-(1,5)-dien wird in einem 1-l-Stahlautoklaven unter Stickstoffatmosphäre innerhalb 3 h auf 145°C erwärmt, wobei der Druck auf ca. $8 \times 10^5$ Pa steigt. Nach 7 h wird auf Raumtemperatur abgekühlt. Nach Abdestillieren von überschüssigem Acetonitril bei ca. $1,5 \times 10^3$ Pa wird eine Probe des entstandenen Öls in Benzylalkohol gelöst und mittels wasserfreier Perchlorsäure titriert. Es wird ein 78%iger Umsatz zu substituierten Pyridinen ermittelt.

Das Rohprodukt wird in halbkonzentrierter Salzsäure gelöst (pH 1), mit ca. dem gleichen Volumen Chloroform versetzt und unter Zusatz von

Aktivkohle 3 h zum Sieden erhitzt. Nach dem Abkühlen wird filtriert und von der Chloroformphase abgetrennt. Die Wasserphase wird mittels Kaliumcarbonat alkalisch gestellt und bei ca. 1,5 × $10^3$ Pa eingedampft. Nach Zusatz von Toluol wird durch azeotrope Destillation bei ca. 2 × $10^3$ Pa restliches Wasser entfernt. Der Rückstand wird dreimal mit je 250 ml Äthanol bei 60 bis 70°C extrahiert. Die verbleibenden Salze werden abfiltriert und die vereinigten Filtrate bei 1,5 × $10^3$ Pa eingeengt. Der Rückstand wird im Hochvakuum destilliert. Sdp. 168-180°C/1,33 × $10^{-3}$ Pa. Ausbeute: 165 g (71,8% d. Th.) Isomerengemisch aus 2,4- und 2,5-Bis(hydroymethyl)-6-methylpyridin.

Der hochviskose Rückstand (40 g) ist eine Mischung aus ca. 30% Produkt (titriert) neben Trishydroxymethylbenzolen. Das Destillat erstarrt beim Stehenlassen zu einer kompakten Kristallmasse; Fp. 70-100°C (Pikrat: Fp. 98-110°C, Picrolonat: Fp. 169-172°C).

Das Isomerenverhältnis wird gaschromatographisch bestimmt (Kapillarsäule: K 20M; 35,4 m): 57% 2,4,6-Isomeres, 43% 2,5,6-Isomeres.

b) *Herstellung unter Rückflussbedingungen.*

Eine Lösung aus 16,4 g (0,4 mol) Acetonitril, 11,2 g (0,2 mol) Propargylalkohol sowie 1,4 g (6,03 mmol Cyclopentadienyl/Kobaltcycloocta-(1,5)-dien wird unter Argonatmosphäre 40 h auf 85°C erhitzt. Man arbeitet auf wie in Beispiel 1a beschrieben und erhält 11,2 g eines Rohprodukts (undestilliert) aus 53% 2,4-Bishydroxymethyl-6-methylpyridin, 38% 2,5-Bishydroxymethyl-6-methylpyridin und 8% Trishydroxymethylbenzolen.

(Gaschromatographische Bestimmung analog Beispiel 1a; Ausbeute an substituierten Pyridinen: 59% d. Th.)

c) *Isomerentrennung.*

535 g des gemäss Beispiel 1a hergestellten (undestillierten) Rohprodukts werden durch Kühlen auf 4°C zur Kristallisation gebracht. Das teilkristalline Gemisch wird in der Kälte mit einer Mischung aus 500 ml Aceton und 100 ml Diäthyläther versetzt und filtriert. Der Filterrückstand wird mit Aceton/Diäthyläther (5:1) nachgewaschen und aus Aceton umkristallisiert. Es werden 54 g weisse Kristalle aus 2,4-Bis(hydroxymethyl)-6-methylpyridin erhalten; Fp. 106-109°C (Pikrat: Fp. 125-128°C).

$^1$H-NMR ($CDCl_3$ + DMSO-$d_6$): δ = 2,38 (s; 3H), 4,47 (s; 2H), 4,53 (s; 2H), 5,23 (s, breit; 2H), 6,90 (s; 1H) und 7,12 ppm (s; 1H)

10 g des gereinigten und vollständig kristallisierten Isomerengemisches (gemäss Beispiel 1a) werden in 600 ml Xylol umkristallisiert. Es werden 0,5 g weisse nadelförmige Kristalle aus 2,5-Bis(hydroxymethyl)-6-methylpyridin erhalten; Fp. 94-96°C.

$^1$H-NMR ($CDCl_3$ + DMSO-$d_6$): δ = 2,37 (s; 3H), 4,46 (s; 2H), 4,51 (s; 2H), 4,88 (s, breit; 2H), 7,12 (d, J = 8Hz; 1H) und 7,59 ppm (d, J = 8Hz; 1H)

d) *Hydrochlorid.*

11 g eines gemäss einem der Beispiele 1a oder 1b hergestellten Isomerengemisches (undestilliertes Rohprodukt) werden bei 20°C mit 150 ml einer äthanolischen HCl-Lösung versetzt. Durch Einengen im Vakuum und Hinzufügen von Aceton wird das Produkt in der Kälte zur Kristallisation gebracht. Es wird abgesaugt, mit Aceton gewaschen und über Phosphorpentoxid getrocknet. Ausbeute: 7,4 g hygroskopischer Kristalle.

*Analyse* für $C_8H_{12}ClNO_2(0,07\ H_2O)$:

| | | | | |
|---|---|---|---|---|
| Ber.: | C 50,67 | H 6,38 | N 7,39 | Cl 18,69 % |
| Gef.: | C 50,40 | H 6,38 | N 7,37 | Cl 18,56 % |

*Beispiel 2:*

*2,4-Bis-(2-hydroxyäthyl)-6-methylpyridin und 2,5-Bis-(2-hydroxyäthyl)-6-methylpyridin*

*Methode A:*

Analog Beispiel 1b werden 21,03 g (0,3 mol) 3-Butin-1-ol, 24,6 g (0,6 mol) Acetonitril und 1,5 g (6,46 mmol) Cyclopentadienyl/Kobaltcycloocta-(1,5)-dien zur Reaktion gebracht. Man arbeitet auf wie unter Beispiel 1a beschrieben und erhält 10,2 g eines öligen Rohproduktes aus 45% 2,4-Bis-(2-hydroxyäthyl)-6-methylpyridin, 35% 2,5-Bis-(2-hydroxyäthyl)-6-methylpyridin und 9% Tris-(2-hydroxyäthyl)benzolen.

(Gaschromatographische Bestimmung analog Beispiel 1a; Ausbeute an substituierten Pyridinen: 30% d. Th.)

*Methode B:*

1,95 g (5,4 mmol) eines Isomerengemisches aus 2,4- bzw. 2,5-Bis-(2-benzyloxyäthyl)-6-methylpyridin, hergestellt gemäss Beispiel 3, werden in 10 ml Trifluoressigsäure gelöst. Nach Zugabe von 0,2 g Palladium/Kohlenstoff (5 Gew.-% Pd) wird bei 20 bis 25°C hydriert. Nach ca. 3 h kommt die Wasserstoffaufnahme (233 ml, 104% d. Th.) zum Stillstand. Nach Abfiltrieren des Katalysators wird im Vakuum vom Lösungsmittel befreit und der ölige Rückstand in 2N-Salzsäure gelöst. Die Lösung wird mit Essigsäureäthylester gewaschen und anschliessend mit Kaliumcarbonat alkalisch gestellt (pH 9). Das Produkt wird mit Essigsäureäthylester extrahiert. Nach Waschen des Extraktes mit gesättigter Kochsalzlösung und Trocknen über Natriumsulfat wird das Lösungsmittel im Vakuum abdestilliert. Es werden 0,9 g eines öligen Produkts erhalten.

$^1$H-NMR ($CDCl_3$): δ = 2,43 und 2,47 (s; 3H), 2,68 bis 2,95 (m; 4H), 3,70 bis 3,95 (m; 4H), 4,08 (s; 2H) und 6,83 bis 7,42 ppm (m; 2H)

b) *2,5-Bis-(2-hydroxyäthyl)-6-methylpyridin.*

9,4 g des gemäss Beispiel 2, Methode A, erhaltenen Produkts werden an einer Kieselgelsäule (6 cm Durchmesser, 1 m Länge) chromatographiert (Elutionsmittel Toluol/Aceton 1:5). Als erste Fraktion (1050 ml) wird eine Lösung erhalten, aus der nach Einengen 0,53 g eines Öls erhalten werden, welches beim Stehenlassen zu Kristallen erstarrt. Die Kristalle werden mit wenig Aceton gewaschen und getrocknet; Fp. 82-84°C.

$^1H$-NMR ($CDCl_3$): δ = 2,48 (s; 3H), 2,73 bis 2,95 (m; 4H), 3,71 bis 3,97 (m; 6H), 6,90 (d, J = 8Hz; 1H) und 7,38 ppm (d, J = 8Hz; 1H)

*Beispiel 3:*

*2,4-Bis-(2-benzyloxyäthyl)-6-methylpyridin und 2,5-Bis-(2-benzyloxyäthyl)-6-methylpyridin*

Analog Beispiel 1b werden 18,4 g (0,115 mol) 4-Benzyloxy-1-butin, 24,6 g (0,6 mol) Acetonitril und 1,5 g (6,46 mmol) Cyclopentadienyl/Kobalt-cycloocta-(1,5)-dien zur Reaktion gebracht. Das Reaktionsgemisch wird mit 200 ml halbkonzentrierter Salzsäure versetzt und die Mischung zweimal mit je 150 ml Essigsäureäthylester extrahiert. Die wässerige Lösung wird mit einer Lösung von 4 g 1-Nitroso-2-naphthol in 200 ml halbkonzentrierter Essigsäure versetzt. Der dabei ausfallende Kobaltkomplex wird abgesaugt. Das Filtrat wird mit 700 ml Diäthyläther gewaschen und anschliessend mit Kaliumcarbonat alkalisch gestellt. Das Produkt wird mit Essigsäureäthylester extrahiert. Nach dem Trocknen über Natriumsulfat und Abziehen des Lösungsmittels im Vakuum werden 2,9 g Rohprodukt erhalten, welches im Kugelrohrofen destilliert wird. Ausbeute: 2,3 g (6%) Öl; Sdp. ca. 190-220°C 0,13 Pa; $n_D^{20}$ = 1,5652.

*Analyse* für $C_{24}H_{27}NO_2$:

| | | | | |
|---|---|---|---|---|
| Ber.: | C 79,74 | H 7,53 | N 3,87 | O 8,85 % |
| Gef.: | C 79,68 | H 7,62 | N 3,94 | O 9,09 % |

*Beispiel 4:*

a) *2,4-Bis(brommethyl)-6-methylpyridin.*

Eine Lösung aus 45 g (0,294 mol) 2,4-Bis(hydroxymethyl)-6-methylpyridin und 160 ml Bromwasserstoffsäure (62%ig) wird 20 h zum Sieden erhitzt. Das Reaktionsgemisch wird anschliessend mit 250 ml Wasser verdünnt und bei 1,5 × $10^3$ Pa eingeengt. Der Rückstand wird in 150 ml Wasser gelöst und die Lösung mit 250 ml Diäthyläther überschichtet. Die Wasserphase wird durch Eintragen von festem Kaliumcarbonat unter starkem Rühren alkalisch gestellt (pH 8). Anschliessend wird die Ätherphase abgetrennt und die Wasserphase mit Diäthyläther nachgewaschen. Die vereinigten Ätherphasen werden über Natriumsulfat getrocknet und bei 1,5 × $10^3$ Pa eingeengt. Es verbleibt ein rotes, viskoses Öl. Ausbeute: 76 g (93% d. Th.). Das Produkt ist bei Raumtemperatur nicht beständig und wird unmittelbar weiterverwendet.

b) *2,4-Bis(brommethyl)-6-methylpyridin und 2,5-Bis(brommethyl)-6-methylpyridin.*

*Methode A:*

Entsprechend Beispiel 4a werden 77,4 g (0,5 mol) des Isomerengemisches gemäss Beispiel 1a mit 270 ml Bromwasserstoffsäure umgesetzt. Es werden 124 g (89% d. Th.) eines öligen Produkts erhalten, welches bei 4°C zu einer halbkristallinen Masse erstarrt; Picrolonat: Fp. 143-144°C.

$^1H$-NMR ($CDCl_3$): δ = 2,50 und 2,57 (s; 3H), 4,28, 4,39 und 4,43 (s; 4H), 7,02 (s), 7,20 (s), 7,20 (d, J = 8Hz) und 7,54 ppm (d, J = 8Hz; zusammen 2H)

*Methode B:*

Entsprechend Beispiel 4a werden 9 g (47 mmol) Hydrochlorid des Isomerengemisches (Beispiel 1d) mit 40 ml Bromwasserstoffsäure umgesetzt. Es werden 9,35 g (71% d. Th.) Produkt erhalten, welches mit dem durch Methode A erhaltenen identisch ist.

*Beispiel 5:*

*[6-Methylpyridin-2,5-diylbis(methylen)]bistriphenylphosphoniumdibromid*

Eine Lösung aus 630 g (2,4 mol) Triphenylphosphin und 1,5 l N,N-Dimethylformamid wird auf 80°C erhitzt. Es wird eine Lösung aus 279 g des Isomerengemisches gemäss Beispiel 4b und 500 ml N,N-Dimethylformamid zugetropft. Nach 3 h Rühren bei 80°C wird auf Raumtemperatur abgekühlt. Im Verlauf einiger Stunden bilden sich feine Kristalle. Diese werden abgesaugt, mit 200 ml kaltem N,N-Dimethylformamid sowie 2 l Diäthyläther gewaschen und aus Wasser umkristallisiert; Ausbeute: 215 g.

*Analyse* für $C_{44}H_{39}NBr_2P_2$:

| | | | | |
|---|---|---|---|---|
| Ber.: | C 65,77 | H 4,89 | N 1,74 | Br 19,89 % |
| Gef.: | C 65,95 | H 4,90 | N 1,76 | Br 19,85 % |

Die Mutterlauge wird bei 1,5× $10^3$ Pa weitgehend vom Lösungsmittel befreit und der Rückstand in Dichlormethan gelöst. Die Lösung wird in Diäthyläther eingerührt, wobei weiteres Produkt als feinpulvriger Niederschlag ausfällt. Ausbeute 479 g Isomerengemisch aus [6-Methylpyridin-2,5-diylbis(methylen)]bistriphenylphosphoniumdibromid und [6-Methylpyridin-2,4-diylbis-(methylen)]bistriphenylphosphoniumdibromid.

$^1H$-NMR ($CDCl_3$): δ = 1,61 und 1,97 (s; 3H), 5,33 und 5,61 (d, J = 15Hz; 4H) und 7,32 bis 8,00 ppm (m, 32 arom. H)

Gesamtausbeute an Triphenylphosphoniumbromiden: 86,4% d. Th.

*Beispiel 6:*

*[6-Methylpyridin-2,4-diylbis(methylen)]bistriphenylphosphoniumdibromid*

Analog Beispiel 5 werden 169 g (0,645 mol) Trisphenylphosphin mit 75 g (0,269 mol) 2,4-Bis-(brommethyl)-6-methylpyridin (nach Beispiel 4a) umgesetzt. Das Produkt wird durch Einrühren der Dichlormethanlösung in Essigsäureäthylester als feinpulvriger Niederschlag isoliert. Ausbeute: 198,3 g (92% d. Th.); Fp. 220-225°C.

*Beispiel 7:*

*[6-Methylpyridin-2,4-diylbis(methylen)]bisphosphonsäuretetraäthylester und [6-Methylpyridin-2,5-diylbis(methylen)]bisphosphonsäuretetraäthylester*

Eine Mischung aus 36,6 g (0,131 mol) der Bisbrommethylpicoline gemäss Beispiel 4b, 47,9 g (0,288 mol) Triäthylphosphit und 140 ml Xylol

wird zum Sieden erhitzt. Unter allmählicher Steigerung der Badtemperatur bis auf 200°C werden die siedenden Bestandteile abdestilliert. Das verbleibende viskose Öl wird bei 50°C/0,13 Pa getrocknet. Ausbeute: 47 g (91% d. Th.); Sdp. ca. 230°C/0,13 Pa (Kugelrohrdestillation).

*Analyse* für $C_{16}H_{29}NO_6P_2$:

| | | | | |
|---|---|---|---|---|
| Ber.: | C 48,85 | H 7,43 | N 3,56 | P 15,75 % |
| Gef.: | C 48,67 | H 7,90 | N 3,89 | P 15,54 % |

*Beispiel 8:*

*2,5-Divinyl-6-methylpyridin*

Zu einer Mischung aus 165 g (0,205 mol) [6-Methylpyridin-2,5-diylbis(methylen)]bistriphenylphosphoniumdibromid (Beispiel 5), 1,2 l wässeriger Formaldehydlösung (38%ig), 1 l Dichlormethan sowie 1,07 g (4 mmol) Tetrabutylammoniumcyanid wird unter schnellem Rühren eine Lösung aus 120 g (3 mol) Natriumhydroxid und 240 ml Wasser innerhalb 1,5 h zugetropft. Dabei wird die Temepratur auf 15°C gehalten. Nach 16 h Rühren bei Raumtemperatur wird die organische Phase abgetrennt und die wässerige Phase mit 200 ml Dichlormethan nachgewaschen. Die vereinigten organischen Phasen werden mit 1,5 l Wasser gewaschen, mit 100 mg Di-tert.-butyl-p-kresol versetzt und bei $1,5 \times 10^3$ Pa eingeengt. Der halbfeste Rückstand wird mit 1,4 l Salzsäure (ca. 10%ig) verrührt. Es wird zweimal mit je 600 ml Essigsäureäthylester extrahiert, und die wässerige Phase mit 100 mg Di-tert.-butyl-p-kresol sowie 700 ml Essigsäureäthylester versetzt. Anschliessend wird mittels festem Kaliumcarbonat alkalisch gestellt und die organische Phase abgetrennt. Nach Trocknen über Natriumsulfat und Einengen bei $1,5 \times 10^3$ Pa wird das flüssige Rohprodukt destilliert; Sdp. 38-40°C/6,7 Pa; Ausbeute 26,5 g (89% d. Th.); $n_D^{20} = 1,5819$.

$^1$H-NMR ($CDCl_3$): δ = 2,59 (s; 3H), 5,33 (m, J = 1,5Hz und 10Hz; 1H), 5,43 (m, J = 1,5Hz und 10Hz; 1H), 5,63 (m, J = 1,5Hz und 18Hz; 1H), 6,15 (m, J = 2,0Hz und 18Hz; 1H), 6,80 (m, J = 10Hz und 18Hz; 1H), 6,89 (m, J = 10Hz und 18Hz; 1H), 7,18 (d, J = 8Hz; 1H) und 7,68 ppm (d, J = 8Hz; 1H)

Das Produkt wird mit Di-tert.-butyl-p-kresol stabilisiert und kühl gelagert.

*Beispiel 9:*

*2,4-Divinyl-6-methylpyridin*

Analog Beispiel 8 werden 165 g (0,205 mol) [6-Methylpyridin-2,4-diylbis(methylen)]bistriphenylphosphoniumdibromid (Beispiel 6) umgesetzt. Ausbeute: 25,3 g (85% d. Th.); Sdp. 45-48°C/9,3 Pa; $n_D^{20} = 1,5682$.

$^1$H-NMR ($CDCl_3$): δ = 2,48 (s; 3H), 5,31 (m, J = 1,5Hz und 10Hz; 1H), 5,36 (m, J = 2Hz und 10Hz; 1H), 5,80 (m, J = 1,5Hz und 18Hz; 1H), 6,12 (m, J = 2Hz und 18Hz; 1H), 6,51 (m, J = 10Hz und 18Hz; 1H), 6,75 (m, J = 10Hz und 18Hz; 1H), 6,88 (s; 1H) und 7,01 ppm (s; 1H)

Das Produkt wird mit Di-tert.-butyl-p-kresol stabilisiert und kühl gelagert.

*Beispiel 10:*

*2,4-Divinyl-6-methylpyridin und 2,5-Divinyl-6-methylpyridin*

*Methode A:*

Analog Beispiel 8 wird das Isomerengemisch der Phosphoniumsalze gemäss Beispiel 5 mit Formaldehyd umgesetzt. Aus 371 g (0,43 mol) Bisphosphoniumsalz mit einem Isomerenverhältnis von 57 Teilen 2,4,6-substituiertem Pyridin zu 43 Teilen 2,5,6-substituiertem Pyridin werden 39,6 (63,5% d. Th.) Divinyl-6-methylpyridine des annähernd gleichen Isomerenverhältnisses gewonnen; Sdp. 40-48°C/20 Pa.

*Methode B:*

3,93 g (0,01 mol) eines Isomerengemisches der Bisphosphonsäuretetraäthylester gemäss Beispiel 7 werden in 70 ml Dichloräthan gelöst. Es werden 35 ml wässerige Formaldehydlösung (35%ig), 35 ml wässerige Natriumhydroxidlösung (50%ig) sowie 0,134 g Tetrabutylammoniumcyanid zugegeben. Des weiteren gibt man je 0,02 g Di-tert.-butyl-p-kresol und Diisopropylxanthogendisulfid als Polymerisationsinhibitoren hinzu.

Den Ansatz wird in einem Stahlautoklaven 4 h auf 100°C erhitzt. Anschliessend wird die organische Phase abgetrennt und die wässerige Phase mit Dichloräthan nachgewaschen. Die vereinigten Extrakte werden unter Zusatz von 10 mg Di-tert.-butyl-p-kresol bei $1,5 \times 10^3$ Pa eingeengt. Der ölige Rückstand wird in halbkonzentrierter Salzsäure gelöst und dreimal mit je 100 ml Essigsäureäthylester gewaschen. Die wässerige Phase wird mit 100 ml Essigsäureäthylester überschichtet und mittels Kaliumcarbonat alkalisch gestellt. Die organische Phase wird abgetrennt, über Natriumsulfat getrocknet und bei $1,5 \times 10^3$ Pa eingeengt. Es werden 2,5 g eines Öls erhalten, welches durch Kugelrohrdestillation in zwei Fraktionen aufgetrennt wird. Die erste Fraktion (Sdp. 70-120°C/27 Pa) enthält 77% (GC) Divinyl-6-methylpyridine, die zweite Fraktion (Sdp. 230°C/0,13 Pa) enthält Ausgangsprodukte sowie [Monovinyl-6-methylpyridinyl(methylen)]phosphonsäurediäthylester.

*Methode C:*

2,48 g (13,6 mmol) des Isomerengemisches aus Bis-(2-hydroxyäthyl)-6-methylpyridinen gemäss Beispiel 2 werden über 2,8 g gepulvertem Kaliumhydroxid bei 1,3 Pa langsam auf 170°C erhitzt. Dabei werden 1,38 g Destillat aufgefangen, welches anschliessend mit 40 ml Diäthyläther versetzt wird. Die Lösung wird über Natriumsulfat getrocknet und eingeengt. Der Rückstand wird über einer kleinen Menge gepulvertem Kaliumhydroxid in Gegenwart von ca. 10 mg Di-tert.-butyl-p-kresol bei 60-70°C/27 Pa redestilliert (Kugelrohrdestillation). Ausbeute: 0,84 g; $n_D^{20} = 1,5721$.

NMR-spektroskopisch wurde ein Isomerenverhältnis von 5 Teilen 2,4,6-substituiertem Pyridin zu 1 Teil 2,5,6-substituiertem Pyridin ermittelt.

*Methode D:*

In einem Stahlautoklaven wird unter Stickstoffatmosphäre eine Mischung aus 164 g (4 mol) Acetonitril, 26 g (0,5 mol) Vinylacetylen und 1,51 g (0,0065 mol) Cyclopentadienyl/Kobalt-cycloocta-(1,5)-dien vorgelegt. Die Lösung wird kontinuierlich durch einen röhrenförmigen Druckreaktor mit ca. 70 ml Fassungsvermögen gepumpt. Durch Thermostatisierung des Reaktors wird eine Temperatur von 148°C gehalten. Die mittlere Verweilzeit des Reaktionsgemisches im Reaktor wird auf 30 min begrenzt. Der Innendruck steigt auf ca. $3,5 \times 10^6$ Pa. Nach weitgehender Entleerung des Reservoirs wird reines Acetonitril eingespeist, um die Reaktionsprodukte vollständig aus dem Reaktor zu entfernen. Es werden 264 g einer Lösung erhalten. Mittels gaschromatographischer Analyse (OV 101,3%) wird ein Hauptprodukt identifiziert, welches mit dem gemäss Methode A hergestellten Isomerengemisch identisch ist. Durch Titration mittels 0,1 N-Perchlorsäure in Dioxan wird eine Ausbeute von 15,6 g (43% d. Th.) an Divinyl-6-methylpyridinen ermittelt.

Die Reaktionsprodukte werden wie folgt isoliert: Das Reaktionsgemisch wird nach Zusatz von ca. 200 mg Di-tert.-butyl-p-kresol bei $1,5 \times 10^3$ Pa und maximal 30°C weitgehend von überschüssigem Acetonitril befreit. Der Rückstand wird in verdünnter Salzsäure gelöst und zweimal mit je 100 ml Essigsäureäthylester gewaschen. Die wässerige Phase wird mit ca. 150 ml Essigsäureäthylester überschichtet und unter Rühren mittels Kaliumcarbonat alkalisch gestellt. Die organische Phase wird abgetrennt, mit ca. 100 mg Di-tert.-butyl-p-kresol versetzt, über Natriumsulfat getrocknet und bei $1,5 \times 10^3$ Pa eingeengt. Das flüssige Rohprodukt wird bei ca. 90°C/27 Pa im Kugelrohr destilliert. Ausbeute an isoliertem Produkt: 9,1 g (25% d. Th.). Mittels NMR-Spektroskopie wird ein Isomerenverhältnis von 17 Teilen 2,4,6-substituiertem Pyridin zu 9 Teilen 2,5,6-substituiertem Pyridin ermittelt.

*Anwendungsbeispiel für das 2,5-Divinyl-6-methylpyridin*

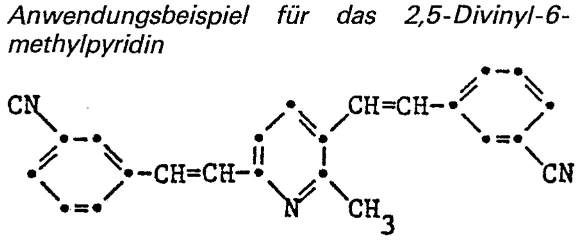

3,64 g (0,02 mol) 3-Brombenzonitril werden in 30 ml N,N-Dimethylformamid und 15 ml Triäthylamin gelöst. Die Lösung wird mit 0,09 g (0,4 mmol) Palladium(II)acetat und 0,25 g (0,8 mmol) Tri-o-tolylphosphin versetzt und anschliessend auf 100°C aufgeheizt. Zu dieser Reaktionsmischung tropft man eine Lösung von 1,45 g (0,01 mol) 2,5-Divinyl-6-methylpyridin. Nach beendeter Zugabe wird noch 2 h bei 100°C gerührt und anschliessend auf Raumtemperatur abgekühlt. Die Reaktionsmischung wird filtriert und das Filtrat mit 70 ml Dimethyläther und 70 ml $H_2O$ versetzt. Aus der organischen Schicht fällt nach Abtrennen des Wassers ein gelbes Kristallisat aus. Nach mehrmaligem Waschen der Kristallmasse mit Toluol und Äther wird das Kristallisat getrocknet. Man erhält 2,0 g gelber Kristalle entsprechend einer Ausbeute von 58% d. Th.

*Analyse* für $C_{24}H_{17}N_3$:

| | | | |
|---|---|---|---|
| Ber.: | C 82,97 | H 4,93 | N 12,10 % |
| Gef.: | C 83,0 | H 5,1 | N 11,9 % |

Die Verbindung hat ein $\lambda_{max.}$ von 362 µm und ein $FL_{max.}$ von 433 µm.

Die Verbindung ist aufgrund der Spektraldaten und der Ausfärbung (Weissgrad) als optischer Aufheller geeignet.

## Patentansprüche

1. Verfahren zur Herstellung von Verbindungen der Formel:

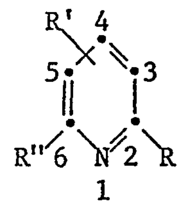

(I)

worin
R' in 4- oder 5-Stellung gebunden ist,
R und R' $-CH_2OH$, $-CH_2CH_2OH$, $-CH_2CH_2OCH_2C_6H_5$, $-CH_2X$, $-CH_2P^+(Y)_3X^-$, $-CH_2P(O)(C_{1-3}\text{-Alkoxy})_2$ oder $-CH=CH_2$,
R'' $C_{1-10}$-Alkyl,
X ein Halogenatom,
$X^-$ das X entsprechende Anion, und
Y Phenyl, das durch eine $C_{1-3}$-Alkylgruppe substituiert sein kann,
bedeuten, dadurch gekennzeichnet, dass man eine Verbindung der Formel:

R''−CN (II)

bei Temperaturen zwischen 40 und 180°C in Gegenwart eines Kobalt(I)-Katalysators mit einer Verbindung der Formel:

HC≡C−Z (III)

zu Verbindungen der Formel:

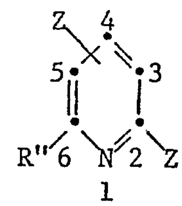

(IV)

umsetzt, wobei R'' die unter Formel (I) angegebene Bedeutung hat und die Z $=CH_2OH$, $-CH_2CH_2OH$ oder $-CH_2CH_2OCH_2C_6H_5$ bedeuten, Verbindungen der Formel (IV), worin die Z $-CH_2CH_2OH$ oder $-CH_2CH_2OCH_2C_6H_5$ darstellen, gegebenenfalls unter vorheriger Hydrierung von Gruppen Z = $-CH_2CH_2OCH_2C_6H_5$ zu Gruppen $-CH_2CH_2OH$, zu Verbindungen der Formel (I) mit R und R' = $-CH=CH_2$ dehydratisiert, und Verbindungen der Formel (IV) mit

$Z = -CH_2OH$ durch Behandlung mit einem Halogenierungsmittel zu Verbindungen der Formel:

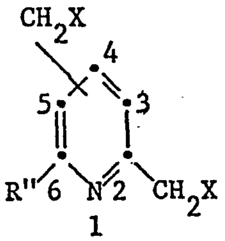

(V)

umsetzt, die Verbindungen der Formel (V) mit einer Verbindung einer der Formeln :

$P(Y)_3$ (VIa) oder $P(O)(C_{1-3}\text{-Alkoxy})_3$ (VIb)

zu Verbindungen der Formeln:

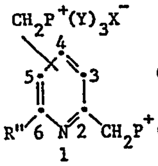

oder

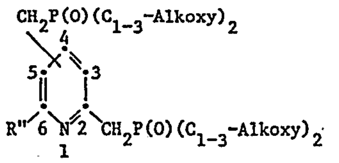

(VIIa) (VIIb)

umsetzt und die Verbindungen einer der Formeln (VIIa) oder (VIIb) in Gegenwart einer Base mit Formaldehyd zu Verbindungen mit R und R' = $-CH{=}CH_2$ umsetzt, wobei R'', Y, X und $X^-$ die unter Formel (I) angegebene Bedeutung haben und die zweite Gruppe Z, $-CH_2$Halogen, $-CH_2P^+(Y)_3X^-$ oder $-CH_2P(O)(C_{1-3}\text{-Alkoxy})_2$ jeweils in 4- oder 5-Stellung an den Pyridinring gebunden ist.

2. Verfahren nach Anspruch 1, dadurch gekennzeichnet, dass man eine Verbindung der Formel (II) verwendet, worin R'' geradkettiges oder verzweigtes $C_{1-4}$-Alkyl, besonders Methyl, darstellt.

3. Verfahren nach Anspruch 1, dadurch gekennzeichnet, dass man eine Verbindung der Formel (III) verwendet, worin Z $-CH_2CH_2OH$ und insbesondere $-CH_2OH$ darstellt.

4. Verfahren nach Anspruch 1, dadurch gekennzeichnet, dass man als Halogenierungsmittel konzentrierte wässerige Chlorwasserstoffsäure und vor allem konzentrierte wässerige Bromwasserstoffsäure verwendet.

5. Verfahren nach Anspruch 1, dadurch gekennzeichnet, dass man als Kobalt(I)-Katalysator Cyclopentadienyl/Kobaltcyclopentadien, das an einer Cyclopentadienylgruppe auch durch $-CH_2CN$, $-CH(CN)-CH_3$, $-C(CN){=}CH_2$ oder $-CH(CN)CH_2OCH_3$ substituiert sein kann, Cyclopentadienyltetraphenyl/Kobaltcyclopentadien, Cyclopentadienyl/Kobaltcyclooctadien, $\eta^3$-Cyclooctadienyl/Kobaltcyclooctadien oder Methylheptadienyl/Kobaltbutadien verwendet.

6. Verfahren nach Anspruch 1, dadurch gekennzeichnet, dass man als Kobalt(I)-Katalysator Cyclopentadienyl/Kobaltcyclooctadien verwendet.

7. Verfahren nach Anspruch 1, dadurch gekennzeichnet, dass man eine Verbindung der Formel (VIa) verwendet, worin Y unsubstituiertes Phenyl darstellt.

8. Verfahren nach Anspruch 1, dadurch gekennzeichnet, dass man eine Verbindung der Formel (VIb) verwendet, worin Alkoxy Äthoxy oder Isopropyloxy bedeutet.

9. Verfahren nach Anspruch 1, dadurch gekennzeichnet, dass man den Kobalt(I)-Katalysator in einer Menge von 0,1 bis 3, besonders 0,1 bis 1 Mol.-%, bezogen auf das Alkin der Formel (III), verwendet.

10. Verfahren nach Anspruch 1, dadurch gekennzeichnet, dass man die Cocyclotrimerisation zu Verbindungen der Formel (IV) bei Temperaturen zwischen 80 und 160°C vornimmt.

11. Verfahren nach Anspruch 1, dadurch gekennzeichnet, dass man einen Überschuss an Nitril der Formel (II), bevorzugt einen 5- bis 20fachen Überschuss über die stöchiometrisch benötigte Menge, verwendet.

12. Verfahren zur Herstellung von Verbindungen der Formel:

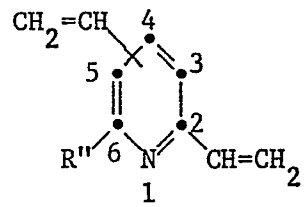

(Ia)

worin die zweite Vinylgruppe in 4- oder 5-Stellung gebunden ist und R'' C-Alkyl bedeutet, dadurch gekennzeichnet, dass man eine Verbindung der Formel:

$R''-CN$ (II)

bei Temperaturen zwischen 30 und 170°C in Gegenwart eines Kobalt(I)-Katalysators mit Vinylacetylen umsetzt.

13. Verbindungen der Formel:

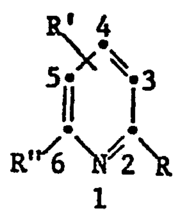

(I)

worin

R' in 4-oder 5-Stellung gebunden ist,

R und R' $-CH_2OH$, $-CH_2CH_2OH$, $-CH_2CH_2OCH_2C_6H_5$, $-CH_2X$, $-CH_2P^+(Y)_3X^-$, $-CH_2P(O)(C_{1-3}\text{-Alkoxy})_2$ oder $-CH{=}CH_2$,

R'' $C_{1-10}$-Alkyl,

X ein Halogenatom,

$X^-$ das X entsprechende Anion, und

Y Phenyl, das durch eine $C_{1-5}$-Alkylgruppe substituiert sein kann,

bedeuten, mit Ausnahme von 2,4-Bis-(β-hydroxyethyl)-6-methylpyridin und 2,4-Divinyl-6-methylpyridin.

## Claims

1. A process for the preparation of a compound of the formula:

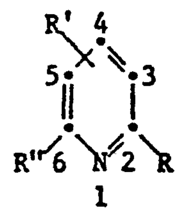

(I)

R' is bound in the 4- or 5-position,
R and R' are $-CH_2OH$, $-CH_2CH_2OH$, $-CH_2CH_2OCH_2C_6H_5$, $-CH_2X$, $-CH_2P^+(Y)_3X^-$, $-CH_2P(O)(C_1\text{-}C_3\text{alkoxy})_2$ or $-CH=CH_2$,
R'' is $C_1$-$C_{10}$alkyl,
X is a halogen atom,
$X^-$ is the anion corresponding to X, and
Y is phenyl which may be substituted by a $C_1$-$C_5$alkyl group,
which process comprises reacting a compound of the formula:

R''−CN (II)

with a compound of the formula:

HC≡C−Z (III)

in the temperature range from 40 to 180°C and in the presence of a cobalt(I) catalyst, to give a compound of the formula:

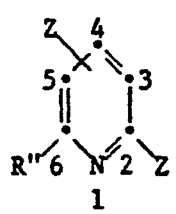

(IV)

wherein R'' is as defined for Formula (I) and the substituents Z are $-CH_2OH$, $-CH_2CH_2OH$ or $-CH_2CH_2OCH_2C_6H_5$,
dehydrating a compound of the Formula (IV), wherein the substituents Z are $-CH_2CH_2OH$ or $-CH_2CH_2OCH_2C_6H_5$, if desired after previously hydrogenating $-CH_2CH_2OCH_2C_6H_5$ groups Z to $-CH_2CH_2OH$ groups, to compounds of the Formula (I), in which R and R' are $-CH=CH_2$,
and reacting a compound of the Formula (IV), in which Z is $-CH_2OH$, by treatment with a halogenating agent, to give a compound of the formula:

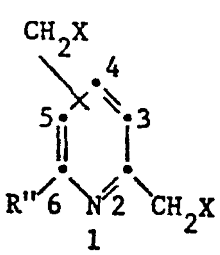

(V)

reacting the compound of the Formula (V) with a compound of the formula:

$P(Y)_3$ (VIa) or $P(O)(C_1\text{-}C_3\text{alkoxy})_3$ (VIb)

to give a compound of the formula:

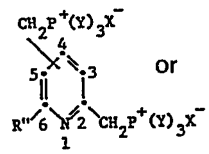

or

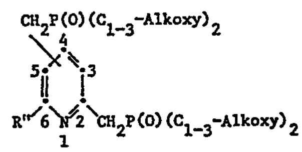

(VIIa) (VIIb)

and reacting the compound of the Formula (VIIa) or (VIIb) with formaldehyde, in the presence of a base, to give a compound in which R and R' are $-CH=CH_2$, and R'', Y, X and $X^-$ are as defined for Formula(I) and the second group Z, $-CH_2$-halogen, $-CH_2P^+(Y)_3X^-$ or $-CH_2P(O)(C_1\text{-}C_3\text{alkoxy})_2$ are each linked to the pyridine ring in the 4- or 5-position.

2. A process according to Claim 1, which comprises the use of a compound of the Formula (II), wherein R'' is straight chain or branched $C_1$-$C_4$alkyl, preferably methyl.

3. A process according to Claim 1, which comprises the use of a compound of the Formula (III), wherein Z is $-CH_2CH_2OH$, or preferably $-CH_2OH$.

4. A process according to Claim 1, wherein the halogenating agent is concentrated aqueous hydrochloric acid, or preferably concentrated aqueous hydrobromic acid.

5. A process according to Claim 1, wherein the cobalt(I) catalyst is cyclopentadienyl/cobalt-cyclopentadiene which may also be substituted at one cyclopentadienyl group by $-CH_2CN$, $-CH(CN)-CH_3$, $-C(CN)=CH_2$ or $-CH(CN)CH_2OCH_3$, or is cyclopentadienyltetraphenyl/cobalt-cyclopentadiene, cyclopentadienyl/cobalt-cyclooctadiene, $\eta^3$-cyclooctadienyl/cobalt-cyclooctadiene or methylheptadienyl/cobalt-butadiene.

6. A process according to Claim 1, wherein the cobalt(I) catalyst is cyclopentadienyl/cobalt-cyclooctadiene.

7. A process according to Claim 1, which comprises the use of a compound of the Formula (VIa), wherein Y is unsubstituted phenyl.

8. A process according to Claim 1, which comprises the use of a compound of the Formula (VIb), wherein alkoxy is ethoxy or isopropyloxy.

9. A process according to Claim 1, wherein the cobalt(I) catalyst is used in an amount of 0.1 to 3 mol-%, preferably 0.1 to 1 mol-%, based on the alkyne of the Formula (III).

10. A process according to Claim 1, wherein the co-cyclotrimerisation to give compounds of the Formula (IV) is carried out in the temperature range from 80 to 160°C.

11. A process according to Claim 1, wherein an excess of nitrile of the Formula (II) is used, preferably a five- to twentyfold excess of the stoichiometrically required amount.

12. A process for the preparation of a compound of the formula:

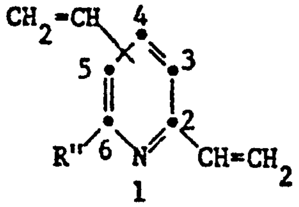

(Ia)

wherein the second vinyl group is bound in the 4- or 5- position and R″ is $C_1$-$C_{10}$alkyl, which process comprises reacting a compound of the formula:

R″−CN (II)

with vinyl acetylene, in the temperature range from 30 to 170°C and in the presence of a cobalt(I) catalyst.

13. A divinylpyridine of Formula (I) substituted in the 6-position and prepared by a process according to Claim 1, with the exception of 2,4-bis-(β-hydroxyethyl)-6-methylpyridine and 2,4-divinyl-6-methylpyridine.

## Revendications

1. Procédé pour préparer des composés répondant à la formule:

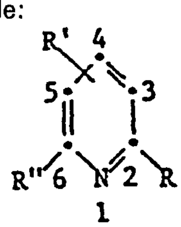

(I)

dans laquelle

R, se trouve en position 4 ou en position 5,

R et R′ représentent chacun un radical $-CH_2OH$, $-CH_2CH_2OH$, $-CH_2CH_2OCH_2C_6H_5$, $-CH_2-X$, $-CH_2P^+(Y)_3X^-$, $-CH_2P(O)(C_{1-3}$-alcoxy$)_2$ ou $-CH{=}CH_2$,

R″ représente un radical alkyle en $C_1$-$C_{10}$,

X représente un atome d'halogène,

$X^-$ représente l'anion correspondant à X, et

Y représente un radical phényle qui peut porter un alkyle en $C_1$-$C_5$,

procédé selon lequel on fait réagir un composé de formule:

R″−CN (II)

à des températures comprises entre 40 et 180°C, en présence d'un catalyseur au coblat(I), avec un composé de formule:

$HC{\equiv}C-Z$ (III)

réaction qui conduit à des composés répondant à la formule:

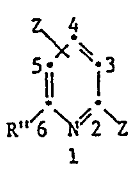

(IV)

dans laquelle R″ a la signification donnée à propos de la formule (I) et les Z représentent chacun un radical $-Ch_2OH$, $-CH_2CH_2OH$ ou $-CH_2CH_2OCH_2C_6H_5$,

on déshydrate des composés de formule (IV) dans lesquels Z représente un radical $-CH_2CH_2OH$ ou $-CH_2CH_2OCH_2C_6H_5$, éventuellement après avoir hydrogéné un radical benzyloxyéthyle ($-CH_2CH_2OCH_2C_6H_5$) Z pour le convertir en un radical $-Ch_2CH_2OH$, déshydratation qui conduit à des composés de formule (I) dans lesquels R et R′ représentent chacun un radical $-CH{=}CH_2$,

on transforme des composés de formule (IV) dans lesquels Z représente un radical $-CH_2OH$, en les traitant par un agent d'halogénation, en composés de formule:

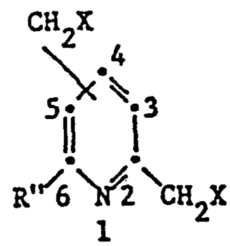

(V)

on fait réagir les composés de formule (V) avec un composé répondant à l'une des formules:

$P(Y)_3$ (VIa) ou $P(O)(C_{1-3}$-alcoxy$)_3$ (VIb)

de manière à obtenir des composés répondant à l'une des formules:

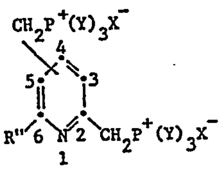

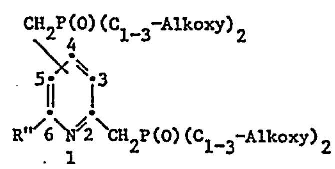

(VIIa) (VIIb)

et on fait réagir les composés de l'une des formules (VIIa) ou (VIIb), en présence d'une base, avec le formaldéhyde de manière à les convertir en composés dans lesquels R et R′ représentent chacun un radical $-CH{=}CH_2$, les symboles R″, Y, X et $X^-$ ayant les significations données ci-dessus à propos de la formule (I) et le second radical Z, qui est un radical halogénométhyle, un radical $-CH_2P^+(Y)_3X^-$ ou un radical $-CH_2P(O)(C_1$-$C_3$-alcoxy$)_2$, occupe à chaque fois la position 4 ou la position 5 sur le noyau de la pyridine.

2. Procédé selon la revendication 1, caractérisé en ce qu'on utilise un composé de formule (II) dans lequel R″ représente un radical alkyle linéaire ou ramifié qui contient de 1 à 4 atomes de carbone, en particulier un radical méthyle.

3. Procédé selon la revendication 1, caractérisé en ce qu'on utilise uncomposé de formule (III) dans lequel Z représente $-CH_2CH_2OH$ ou, plus particulièrement, $-CH_2OH$.

4. Procédé selon la revendication 1, caractérisé en ce qu'on utilise, comme agent d'halogénation, un acide chlorhydrique aqueux concentré ou,

mieux encore, un acide bromhydrique aqueux concentré.

5. Procédé selon la revendication 1, caractérisé en ce qu'on utilise, comme catalyseur au cobalt(I), le cyclopentadiényl/cobaltcyclopentadiène, lequel peut porter un radical $-CH_2CN$, $-CH(CN)-CH_3$, $-C(CN)=CH_2$ ou $-CH(CN)CH_2OCH_3$ sur son radical cyclopentadiényle, le cyclopentadiényltétraphényl/cobaltcyclopentadiène, le cyclopentadiényl/cobaltcyclooctadiène, $\eta^3$-cyclooctandiényl/cobaltcyclooctadiène ou le méthylheptadiényl/cobaltbutadiène.

6. Procédé selon la revendication 1, caractérisé en ce qu'on utilise, comme catalyseur au cobalt(I), le cyclopentadiényl/cobaltcyclooctadiène.

7. Procédé selon la revendication 1, caractérisé en ce qu'on utilise un composé de formule (VIa) dans lequel Y représente un radical phényle non substitué.

8. Procédé selon la revendication 1, caractérisé en ce qu'on utilise un composé de formule (VIb) dans lequel le radical alcoxy est un radical éthoxy ou isopropyloxy.

9. Procédé selon la revendication 1, caractérisé en ce qu'on utilise le catalyseur au cobalt(I) en une quantité de 0,1 à 3% en mol, en particulier de 0,1 à 1% en mol, par rapport à l'alcyne de formule (III).

10. Procédé selon la revendication 1, caractérisé en ce que la cocyclotrimérisation conduisant à des composés de formule (IV) est effectuée à des températures comprises entre 80 et 160°C.

11. Procédé selon la revendication 1, caractérisé en ce qu'on utilise un excès de nitrile de formule (II), de préférence un excès de 5 à 20 fois au-dessus de la quantité stœchiométrique.

12. Procédé pour préparer des composés répondant à la formule:

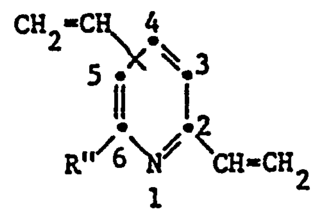

(Ia)

dans laquelle le second radical vinyle se trouve en position 4 ou en position 5 et R" représente un alkyle en $C_1$-$C_{10}$, procédé caractérisé en ce qu'on fait réagir un composé de formule:

R"−CN (II)

avec le vinylacétylène à des températures comprises entre 30 et 170°C et en présence d'un catalyseur au cobalt(I).

13. Divinylpyridines substituées en position 6 qui répondent à la formule (I) et qui ont été préparées par le procédé de la revendication 1, à l'exception de la bis(hydroxy-2 éthyl)-2,4 méthyl-6 pyridine et de la divinyl-2,4 méthyl-6 pyridine.